# EUROPÄISCHE PATENTSCHRIFT

(11) **EP 0 490 200 B1**
(45) Veröffentlichungstag und Bekanntmachung des Hinweises auf die Patenterteilung: **28.02.1996**
(21) Anmeldenummer: 91120615.9
(22) Anmeldetag: 29.11.1991
(51) Int. Cl.: C07D 215/50, B41M 5/136

(54) **Derivate von Chinolincarbonsäuren**
Derivatives of quinoline carboxylic acid
Dérivés d'acide carboxylique de quinoléine

(30) Priorität: 10.12.1990 DE 4039298
(43) Veröffentlichungstag der Anmeldung: 17.06.1992
(73) Patentinhaber: BASF Aktiengesellschaft, D-67063 Ludwigshafen (DE)
(72) Erfinder: Schloesser, Ulrike, Dr., W-6800 Mannheim 81 (DE); Wagenblast, Gerhard, Dr., W-6719 Weisenheim (DE)

(56) Entgegenhaltungen:
- EP-A- 0 256 180
- EP-A- 0 384 313
- DE-A- 3 405 395

## Beschreibung

Die vorliegende Erfindung betrifft neue Chinolincarbonsäurederivate der Formel I 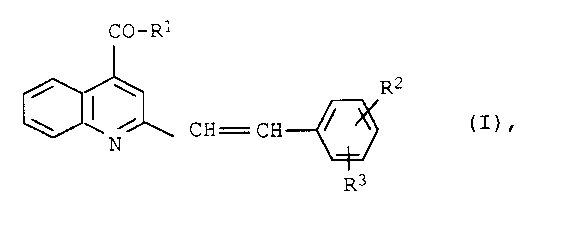
in der
- R¹: C₁-C₈-Alkoxy oder C₁-C₄-Mono- oder Dialkylamino und
einer der beiden Reste R² und R³ C₁-C₄-Alkoxy und der andere Wasserstoff bedeuten,
sowie ihre Verwendung als Farbbildner zur Herstellung von druckempfindlichen Aufzeichnungspapieren.

Aus der DE-A-440 008 sind der 2-Styrylchinolin-4-carbonsäuremethyl- und -ethylester bekannt. Sie sollen dort als Bakterizide verwendet werden. Auch in Chem. Heterocycl. Comp. Vol. 24, 670, 1988, werden diese Verbindungen beschrieben. Auf eine Verwendung als Farbbildner wird nicht hingewiesen.

Aus der DE-A-3 405 395 sind Farbbildner auf Chinolinbasis bekannt. Es handelt sich dabei um 2-Styrylchinolinderivate, die in Ringposition 4 des Chinolinrings keine Substituenten aufweisen.

In der EP-A-256 180 sind 2-Styrylchinolinderivate beschrieben, die im Chinolinring keine Substituenten und im Styrylrest zwei Alkoxygruppen enthalten.

Außerdem beschreiben die EP-A-339 518 sowie die EP-A-384 313 Chinolinderivate, die in Ringposition 2 eine substituierte Phenylgruppe aufweisen und als Farbbildner Anwendung finden können. Es hat sich jedoch gezeigt, daß die Produkte des Standes der Technik noch anwendungstechnische Mängel besitzen.

Aufgabe der vorliegenden Erfindung war es nun, neue Chinolincarbonsäurederivate bereitzustellen, die über vorteilhafte anwendungstechnische Eigenschaften verfügen.

Demgemäß wurden die eingangs näher bezeichneten Chinolincarbonsäurederivate der Formel I gefunden.

Alle in der obengenannten Formel I auftretenden Alkylgruppen können sowohl geradkettig als auch verzweigt sein.

Reste R¹, R² und R³ sind beispielsweise Methoxy, Ethoxy, Propoxy, Isopropoxy, Butoxy, Isobutoxy, sec-Butoxy oder tert-Butoxy.

Reste R¹ sind weiterhin z. B. Pentyloxy, Isopentyloxy, Neopentyloxy, tert-Pentyloxy, Hexyloxy, 2-Methylpentyloxy, Heptyloxy, Octyloxy, 2-Ethylhexyloxy, Isooctyloxy (Die Bezeichnung Isooctyl ist eine Trivialbezeichnung und stammt von den nach der Oxosynthese erhaltenen Alkoholen (vgl. dazu Ullmanns Encyclopädie der technischen Chemie, 4. Auflage, Band 7, Seite 215, sowie Band 11, Seiten 435 und 436.), Mono- oder Dimethylamino, Mono- oder Diethylamino, Mono- oder Dipropylamino, Mono- oder Diisopropylamino, Mono- oder Dibutylamino, Mono- oder Diisobutylamino, oder Mono- oder Di(sec-butyl)amino.

Die neuen Chinolinderivate der Formel I können nach an sich bekannten Methoden, wie sie beispielsweise in Chem. Heterocycl. Comp. Vol. 24, 670, 1988, beschrieben sind, erhalten werden.

Beispielsweise kann man Isatin der Formel II 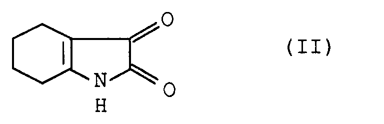
mit Aceton in Gegenwart einer Base in 2-Methylchinolincarbonsäure der Formel III 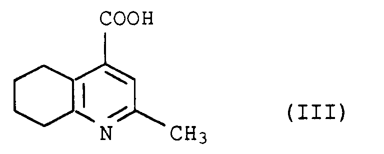
überführen.

Durch Kondensation von Benzaldehyden der Formel IV 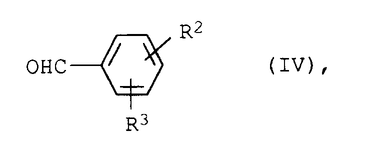
in der R² und R³ jeweils die obengenannte Bedeutung besitzen, mit der Methylchinolincarbonsäure III in saurem Medium gelangt man zu den Styrylchinolincarbonsäuren der Formel V 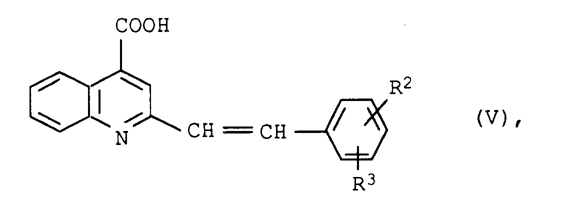
in der R² und R³ jeweils die obengenannte Bedeutung besitzen. Diese können dann beispielsweise in die entsprechenden Carbonsäurehalogenide umgewandelt und mit Verbindungen der Formel VI

R¹ - H (VI),

in der R¹ die obengenannte Bedeutung besitzt, in die Chinolincarbonsäurederivate der Formel I übergeführt werden.

Die vorliegende Erfindung betrifft weiterhin die Verwendung der Chinolincarbonsäurederivate der Formel I als Farbbildner zur Herstellung von druckempfindlichen Aufzeichnungspapieren.

Die Chinolincarbonsäurederivate der Formel I sind schwach farbige bis farblose Verbindungen, deren Lösungen in inerten organischen Lösungsmitteln im Kontakt mit Elektronenacceptoren, je nach der Substitution des Chinolincarbonsäurederivates, Färbungen in grüngelben bis orangeroten Farbtönen geben. Beispiele für Elektronenacceptorsubstanzen sind Carbon- oder Mineralsäuren, Kaolin, Bentonit, aktivierter Ton, Aluminiumsilikat, Attapulgit oder jeder beliebige Ton, sauer reagierende polymere Materialien, wie Kondensationsprodukte auf der Basis von Phenolen und/oder Phenolsulfonsäuren, ferner Metalloxide oder -salze, wie Zinkoxid, Aluminiumoxid, Zinkchlorid, Eisenstearat oder Cobaltnaphthenat.

Aufgrund dieser Eigenschaften sind die Verbindungen der Formel I als Farbbildner zur Verwendung in druckempfindlichen Aufzeichnungsmaterialien geeignet.

Für die Anwendung in druckempfindlichen Systemen werden die Chinolincarbonsäurederivate Ia vorteilhaft in Form von Lösungen in organischen Lösungsmitteln, z.B. Chlorparaffine, partiell hydriertes Di- oder Terphenyl, Alkylbenzole, Alkylnaphthaline, alkylierte Dibenzylbenzole, Paraffinöl, Mineralöl oder auch übliche tiefer siedende Lösungsmittel, wie Xylol oder Toluol, in Mikrokapseln eingeschlossen und mit diesen der Träger, z.B. Papier beschichtet. Bei Druck tritt dann im Kontakt mit Elektronenacceptoren an der Druckstelle Farbbildung ein.

Geeignete Verfahren zur Herstellung von Mikrokapseln sind z.B. aus der US-A-2 800 457, US-A-2 800 458, DE-A-2 119 933 oder EP-A-26 914 bekannt. Man kann die erfindinngsgemäßen Verbindungen auch nach dem in der US-A-3 103 404 beschriebenen Verfahren in Wachs oder Öl-Wachsmischungen fein verteilen und mit diesen Mischungen Träger, wie Folien oder Papier, beschichten. Man erhält druckempfindliche Materialien, die zum Durchschreiben auf mit Elektronenacceptorsubstanzen beschichteten Papieren geeignet sind und die nach Gebrauch wie Kohlepapier entfernt werden.

Die folgenden Beispiele sollen die Erfindung näher erläutern.

### Beispiel 1

### 1. Stufe: 2-Methylchinolin-4-carbonsäure

287 g (1,6 mol) feuchtes Isatin (Trockengehalt: 82 Gew.%) wurden in 2000 ml 38 gew.%iger Natronlauge suspendiert, mit 25,4 g (0,64 mol) Natriumhydroxid und 1,1 g eines Netzmittels versetzt und auf 50°C erwärmt. Bei dieser Temperatur erfolgte innerhalb von 15 Minuten die Zugabe von 93 g (1,6 mol) Aceton. Es wurde 3 Stunden bei 50°C nachgerührt, dann auf 25°C abgekühlt und das feste Produkt abgesaugt. Nach dem Waschen mit 33 gew.%iger Natronlauge wurde der Rückstand in 2,5 l Wasser gelöst, mit 30 gew.%iger Salzsäure bei einem pH-Wert von 2 wieder gefällt, abgesaugt und mit salzsaurem Wasser (pH 2) gewaschen.

Nach dem Trocknen bei 60°C resultierten 219 g (73 %) 2-Methylchinolin-4-carbonsäure vom Schmp. 247-249°C.

### 2. Stufe: 2-(4-Methoxystyryl)chinolin-4-carbonsäure

150 g (0,8 mol) 2-Methylchinolin-4-carbonsäure und 110 g (0,8 mol) p-Methoxybenzaldehyd wurden in 300 ml Eisessig 6 Stunden zum Sieden erhitzt, wobei gleichzeitig 73 g Eisessig abdestilliert wurden. Die Isolierung und Reinigung der Carbonsäure erfolgte durch Absaugen und Waschen des Nutschgutes mit Eisessig und Methanol. Nach dem Trocknen verblieben 144 g (59 %) 2-(4-Methoxystyryl)chinolin-4-carbonsäure vom Schmp. 284-286°C.

### 3. Stufe: 2-(4-Methoxystyryl)chinolin-4-carbonsäuremethylester

12,3 g (0,04 mol) 2-(4-Methoxystyryl)chinolin-4-carbonsäure in 50 ml Thionylchlorid und 1 ml Pyridin wurden für 90 Minuten auf 30°C und für weitere 15 Minuten auf 60°C erwärmt. Das überschüssige Thionylchlorid wurde unter vermindertem Druck entfernt und das Säurechlorid durch Zugabe von 150 ml Methanol in den 2-(4-Methoxystyryl)chinolin-4-carbonsäure methylester übergeführt. Nach weiteren 12 Stunden stehenlassen bei Raumtemperatur wurde das als Hydrochlorid ausgefallene Produkt abgesaugt und mit Methanol gewaschen. Durch Suspendieren und Rühren des Rohproduktes in wäßriger Bicarbonatlösung bis sich die anfänglich rote Masse gelb färbte, erfolgte die Freisetzung der Base.

Die Ausbeute an 2-(4-Methoxystyryl)chinolin-4-carbonsäuremethylester der Formel 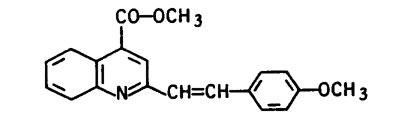
betrug nach dem Absaugen, Neutralwaschen und Trocknen 7,2 g (56 %). Schmp. 99,5°C.
λₘₐₓ (gemessen in Dichlormethan): 373 nm
λₘₐₓ (gemessen in Methanol, das 2 Gew.% Chlorwasserstoff enthielt): 432 nm

### Beispiel 2

### 2-(4-Methoxystyryl)chinolin-4-carbonsäuredi(n-butyl)amid

12,3 g (0,04 mol) 2-(4-Methoxystyryl)chinolin-4-carbonsäure (siehe Bsp. 1) wurden bei maximal 30°C in 50 ml Thionylchlorid unter Rühren gelöst und mit 1 ml Pyridin versetzt. Nach 1,75 Stunden wurde das überschüssige Thionylchlorid unter vermindertem Druck entfernt und der Rückstand unter Eiskühlung mit 150 ml Di-n-butylamin versetzt, wobei die Reaktionstemperatur bis 80°C anstieg. Die Reaktionsmischung wurde weitere 12 Stunden bei Raumtemperatur gerührt und dann in 500 ml Wasser eingetragen. Die Reinigung der organischen Phase erfolgte durch Ausrühren mit 5 gew.%iger Salzsäure und Absaugen des anfallenden Rückstandes. Zur weiteren Reinigung wurde in Toluol aufgenommen und dann mit 5 gew.%iger Salzsäure und verdünnter Natronlauge gewaschen.

Das nach dem Trocknen über Natriumsulfat und Entfernen des Lösungsmittels resultierende Öl wurde durch Filtration über Kieselgel mit Toluol/Essigester (4:1 v/v) als Eluens gereinigt. Die Kristallisation aus Cyclohexan lieferte 4,6 g (27 %) 2-(4-Methoxystyryl)chinolin-4-carbonsäuredi(n-butyl)amid der Formel 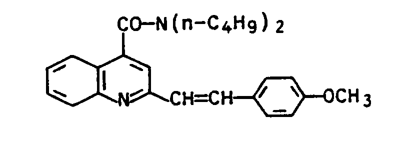
vom Schmp. 77°C.
λₘₐₓ (gemessen in Dichlormethan): 362 nm
λₘₐₓ (gemessen in Methanol, das 2 Gew.% Chlorwasserstoff enthielt): 420 nm

Analog den Beispielen 1 und 2 wurden die in der folgenden Tabelle aufgeführten Chinoline der Formel 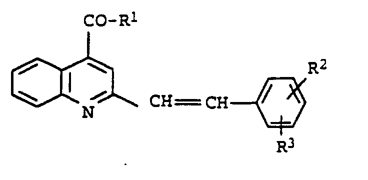
erhalten.

**Tabelle**

| Nr. | R¹ | R² | R³ | Schmp. [°C] | λ max*) [nm] | λmax**) [nm] |
|---|---|---|---|---|---|---|
| 3 | O-nC₄H₉ | H | 4-OCH₃ | 90 | 372 | 432 |
| 4 | O-nC₄H₉ | H | 4-nOC₄H₉ | 81 | 374 | 436 |
| 5 | O-nC₈H₁₇ | H | 4-OCH₃ | 59 | 372 | 432 |
| 6 | O-nC₄H₉ | H | 2-nOC₄H₉ | 56 | 367 | 424 |
| 7 | N-nC₈H₁₇ | H | 4-OCH₃ | 131 | 377 | 415 |

| | | | | | | |
|---|---|---|---|---|---|---|
| *) gemessen in Dichlormethan | | | | | | |
| **) gemessen in Methanol, das 2 Gew.% Chlorwasserstoff enthielt | | | | | | |

### Beispiel 8 (Anwendung)

Eine 0,5 gew.%ige Lösung des Chinolincarbonsäurederivats aus Beispiel 1 in Diisopropylnaphthalin wurde mit einer 6 µm-Rakel auf ein CF-Papier der Fa. Wiggins Teape aufgetragen. Es entwickelte sich ein gelber Farbton, dessen K/S-Wert (Farbstärke) nach dem CIELAB-System bestimmt wurde. Nach einstündigem Belichten im Suntest-Gerät der Fa. Hanau wurde der ΔE-Wert (Lichtechtheit) ebenfalls gemäß CIELAB ermittelt.
K/S : 2,4 ΔE : 8

## Patentansprüche

1. Chinolincarbonsäurederivate der Formel I 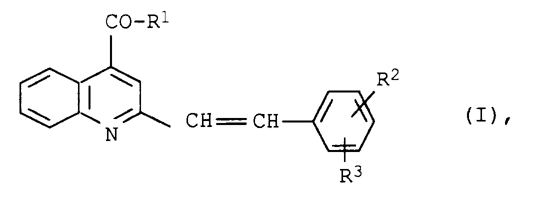 in der
R¹ C₁-C₈-Alkoxy oder C₁-C₄-Mono- oder Dialkylamino und
einer der beiden Reste R² und R³ C₁-C₄-Alkoxy und der andere Wasserstoff bedeuten.

2. Verwendung der Chinolincarbonsäurederivate gemäß Anspruch 1 als Farbbildner zur Herstellung von druckempfindlichen Aufzeichnungspapieren.

## Claims

1. Quinolinecarboxylic acid derivatives of the formula I 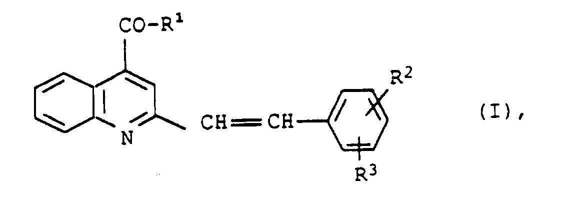 where
R¹ is C₁-C₈-alkoxy or mono- or di(C₁-C₄-alkyl)amino, and
one of R² and R³ is C₁-C₄-alkoxy and the other is hydrogen.

2. The use of the quinolinecarboxylic acid derivatives of claim 1 as color formers in pressure-sensitive recording papers.

## Revendications

1. Dérivés d'acide quinoléinecarboxylique de formule I 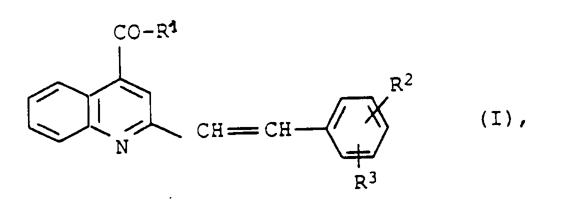 dans laquelle
R¹ représente un groupement alcoxy en C₁-C₈ ou un groupement mono- ou dialkylamino en C₁-C₄ et
l'un des deux restes R² et R³ représente un groupement alcoxy en C₁-C₄ et l'autre un atome d'hydrogène.

2. Utilisation des dérivés d'acide quinoléinecarboxyliques selon la revendication 1 comme chromogènes pour la fabrication de papiers d'enregistrement sensibles à la pression.
